# EUROPEAN PATENT APPLICATION

(11) **EP 4 803 500 A1**
(43) Date of publication of application: **09.09.2026**
(21) Application number: 26162012.4
(22) Date of filing: 03.03.2026
(51) Int. Cl.: C07C 29/151, C07C 31/04

(54) **METHANOL PRODUCTION SYSTEM THAT EFFICIENTLY RECYCLES UNREACTED GAS THROUGH INTERMEDIATE COOLING DISCHARGE FUNCTION AND HYDROGEN RECOVERY**

(30) Priority: 04.03.2025 KR 20250027709
(71) Applicant: Doosan Enerbility Co., Ltd., Seongsan-gu Changwon-si, Gyeongsangnam-do 51711 (KR)
(72) Inventor: Kim, Bong Keun, 16814 Yongin-si, Gyeonggi-do (KR); Bae, Seong Hee, 18475 Hwaseong-si, Gyeonggi-do (KR); Kim, Hyun Min, 16868 Yongin-si, Gyeonggi-do (KR); Nam, Gyeong Mo, 16821 Yongin-si, Gyeonggi-do (KR); Kim, You Seok, 16923 Yongin-si, Gyeonggi-do (KR); Sohn, Geun, 13607 Seongnam-si, Gyeonggi-do (KR)
(74) Representative: BCKIP Part mbB

(57) **Abstract**

A methanol production system capable of improving methanol production efficiency is provided. The methanol production system includes a waste gas reformer configured to reform a waste gas comprising a hydrocarbon (CxHy) gas and carbon dioxide to generate a synthesis gas containing hydrogen, carbon monoxide, and carbon dioxide; a methanol production reactor including a first reaction zone containing a first catalyst, the first reaction zone configured to receive the synthesis gas and facilitate a reaction of the synthesis gas to generate methanol, and a second reaction zone containing a second catalyst, the second reaction zone configured to receive unreacted gas from the first reaction zone and facilitate a reaction of the unreacted gas to generate methanol; a first condenser configured to condense and separate methanol and water from gas discharged from the methanol production reactor; and a gas separator configured to separate a recycle gas comprising hydrogen, or a mixture of hydrogen and carbon monoxide, from gas discharged from the first condenser.

## Description

This application claims priority to Korea Patent Application No. 10-2025-0027709, filed on March 04, 2025.

### FIELD

Apparatuses and methods consistent with exemplary embodiments relate to a methanol production system, and more particularly, to a methanol production system configured to increase methanol production efficiency by utilizing a synthesis gas including carbon monoxide, hydrogen, and a high-concentration carbon dioxide, and by separating and recycling hydrogen following the methanol production reaction.

### BACKGROUND

In a related art, a synthesis gas containing carbon monoxide and hydrogen is supplied to a methanol production reactor, and methanol is produced in the reactor via a catalytic reaction of carbon monoxide and hydrogen.

In the related art systems, carbon dioxide levels in the synthesis gas supplied to the reactor have to be restricted to a low level, for example, below 5%. When carbon dioxide is contained in the synthesis gas, an additional process is necessitated to separate and remove the carbon dioxide before the synthesis gas is introduced into the reactor. In other words, only carbon monoxide and hydrogen are selectively supplied from the synthesis gas to the reactor.

Aspects of one or more exemplary embodiments provide a methanol production system capable of improving methanol production efficiency by utilizing synthesis gas including carbon monoxide, hydrogen, and high-concentration carbon dioxide, and by separating and recycling hydrogen following the methanol production reaction.

Additional aspects will be set forth in part in the description which follows and, in part, will become apparent from the description, or may be learned by practice of the exemplary embodiments.

According to an aspect of an exemplary embodiment, there is provided a methanol production system including: a waste gas reformer configured to reform a waste gas comprising a hydrocarbon (CxHy) gas and carbon dioxide to generate a synthesis gas containing hydrogen, carbon monoxide, and carbon dioxide; a methanol production reactor including a first reaction zone containing a first catalyst, the first reaction zone configured to receive the synthesis gas and facilitate a reaction of the synthesis gas to generate methanol, and a second reaction zone containing a second catalyst, the second reaction zone configured to receive unreacted gas from the first reaction zone and facilitate a reaction of the unreacted gas to generate methanol; a first condenser configured to condense and separate methanol and water from gas discharged from the methanol production reactor; and a gas separator configured to separate a recycle gas comprising hydrogen, or a mixture of hydrogen and carbon monoxide, from gas discharged from the first condenser.

The gas separator may include a pressure swing adsorption (PSA) apparatus or a hydrogen-selective membrane configured to separate hydrogen, and the hydrogen separated in the gas separator may be recycled for methanol production as the recycle gas.

The gas separator may include a carbon dioxide-selective membrane configured to separate carbon dioxide, and hydrogen and carbon monoxide remaining after the separation of carbon dioxide by the gas separator may be recycled for methanol production as the recycle gas.

The recycle gas separated in the gas separator may be merged with the synthesis gas supplied to the first reaction zone.

A portion of the recycle gas separated in the gas separator may be merged with the unreacted gas supplied to the second reaction zone.

The methanol production system may further include a second condenser configured to condense methanol and water from gas discharged from the first reaction zone, and only the unreacted gas discharged from the second condenser after methanol and water are condensed and separated may be supplied to the second reaction zone.

The methanol production system may further include a first compressor configured to compress the synthesis gas and deliver the compressed synthesis gas to the first reaction zone, and the first compressor may be configured to pressurize the synthesis gas to a pressure within a range of approximately 20 bar to 100 bar.

The methanol production system may further include a second compressor configured to compress the recycle gas separated by the gas separator and merge the compressed recycle gas with the synthesis gas supplied to the first reaction zone.

The methanol production system may further include a heat exchanger configured to heat the synthesis gas and supply the heated synthesis gas to the first reaction zone, and the synthesis gas may be heated to a temperature within a range of approximately 150°C to 300°C by the heat exchanger.

The heat exchanger may use the gas discharged from the first reaction zone or the second reaction zone as a heat source.

The recycle gas separated by the gas separator may be merged with the synthesis gas at a position upstream of the heat exchanger.

A portion of the gas discharged from the first condenser after methanol and water are condensed and separated may be branched and supplied to the gas separator, and the methanol production system may further include a flow control valve configured to control a flow rate of the gas branched and supplied to the gas separator.

The methanol production reactor may be configured as a single reactor in which the first reaction zone and the second reaction zone are disposed therein.

The waste gas supplied to the waste gas reformer may have a molar ratio of methane to carbon dioxide of 7:3, or a methane molar ratio lower than 7:3.

The synthesis gas generated by the waste gas reformer may include 0% to 70% hydrogen, 0% to 50% carbon monoxide, 0% to 20% carbon dioxide, and 5% or less methane.

According to one or more exemplary embodiments, the system is configured to produce methanol using synthesis gas containing carbon monoxide, hydrogen, and high-concentration carbon dioxide. Because both carbon monoxide and carbon dioxide serve as reactive species with hydrogen to produce methanol within a single reactor, methanol can be produced under high carbon dioxide concentration conditions without pre-reaction separation or removal of carbon dioxide from synthesis gas generated by reforming waste gas such as biogas or pyrolysis gas.

In addition, the system is configured to separate hydrogen, or a mixture of hydrogen and carbon monoxide, as a recycle gas following the methanol production reaction. By recycling the recycle gas for methanol production, the methanol production efficiency can be improved. Specifically, by using the recycle gas, a methanol synthesis ratio R((H₂-CO₂)/(CO+CO₂)) of the synthesis gas supplied to the first reaction zone can be adjusted within a range of 1.5 to 2.5, thereby maximizing methanol production efficiency.

Furthermore, by condensing and separating methanol and water as intermediate products from the gas discharged from the first reaction zone and supplying only unreacted gas to the second reaction zone, the reaction efficiency of the second reaction zone can be improved, and the volume of the second reaction zone can be reduced relative to the first reaction zone.

### BRIEF DESCRIPTION OF THE DRAWINGS

The above and other will be more clearly understood from the following description of the exemplary embodiments with reference to the accompanying drawings, in which:
FIG. 1 is a schematic diagram schematically illustrating a methanol production system according to a first exemplary embodiment;
FIG. 2 is a schematic diagram schematically illustrating a methanol production system according to a second exemplary embodiment; and
FIG. 3 is a schematic diagram schematically illustrating a methanol production system according to a third exemplary embodiment.

### DETAILED DESCRIPTION

Various modifications and various embodiments will be described with reference to accompanying drawings so that those skilled in the art can easily carry out the disclosure. It should be understood, however, that the various embodiments are not for limiting the scope of the disclosure to the specific embodiments, but they should be interpreted to include all modifications, equivalents or alternatives of the embodiments included within the spirit and scope disclosed herein.

The terms described below are for the purpose of describing specific embodiments only, and are not intended to limit the scope of the present disclosure. As used herein, the singular forms "a", "an" and "the" are intended to include the plural forms as well, unless the context clearly indicates otherwise. In the disclosure, terms such as "comprises", "includes", or "have/has" should be construed as designating that there are such features, integers, steps, operations, components, parts, and/or combinations thereof, and do not exclude the presence or possibility of adding of one or more of other features, integers, steps, operations, components, parts, and/or combinations thereof.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

Terms such as "first," "second," and so on may be used to describe a variety of elements, but the elements should not be limited by these terms. The terms are used simply to distinguish one element from other elements. The use of such ordinal numbers should not be construed as limiting the meaning of the term. For example, the components associated with such an ordinal number should not be limited in the order of use, placement order, or the like. If necessary, each ordinal number may be used interchangeably.

The terms, such as 'part' or 'module', etc., should be understood as a unit that performs at least one function or operation and that may be embodied as hardware, software, or a combination thereof. With respect to an element described as a "unit" or "module", two or more elements may be combined into a single element, or a single element may be divided into two or more elements according to the subdivided functions. Also, each element described below may additionally perform some or all of functions performed by other elements, in addition to its main functions, and some of the main functions of each element may be performed entirely by other components.

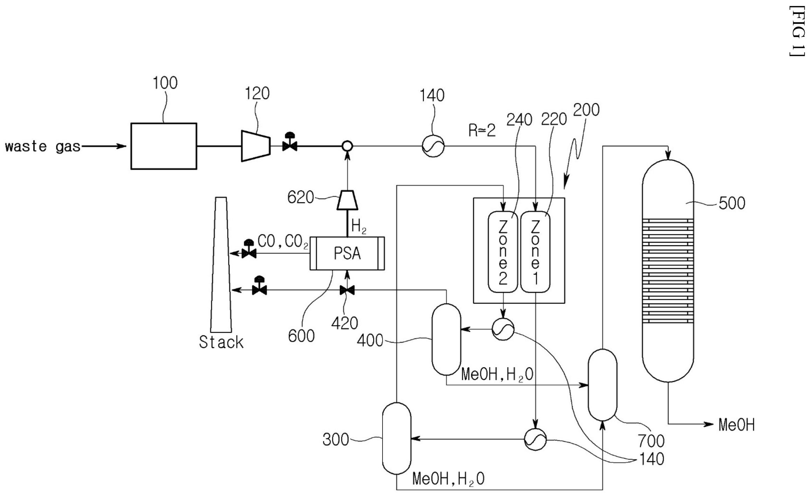

Hereinafter, a methanol production system according to a first exemplary embodiment will be described in detail with reference to FIG. 1.

Referring to FIG. 1, the methanol production system includes a waste gas reformer 100, a first compressor 120, a heat exchanger 140, a methanol production reactor 200, a second condenser 300, a first condenser 400, a distillation column 500, a gas separator 600, and a second compressor 620.

The waste gas reformer 100 reforms waste gas, which is a hydrocarbon (CxHy) gas containing carbon dioxide, to generate a synthesis gas containing hydrogen, carbon monoxide, and carbon dioxide. The waste gas may comprise any gas containing carbon dioxide and hydrocarbons (CxHy), such as biogas or pyrolysis gas. For example, the waste gas introduced into the waste gas reformer 100 has a molar ratio of methane to carbon dioxide of approximately 7:3, or a lower methane molar ratio, such as 6.5:3.5.

More specifically, the waste gas reformer 100 directly reforms the waste gas into synthesis gas by sequentially passing the waste gas through a first reformer in which the waste gas is reacted and reformed at a first temperature, and a second reformer in which the waste gas is reacted and reformed at a second temperature higher than the first temperature. For example, when the waste gas is pyrolysis gas containing carbon dioxide and higher hydrocarbons having two or more carbon atoms, the higher hydrocarbons may react with steam in the first reformer to form methane, and the methane may react with steam in the second reformer to form synthesis gas. In another example, when the waste gas is biogas containing carbon dioxide and methane, the methane may react with steam in the first reformer to form synthesis gas, and the methane may react with carbon dioxide in the second reformer to form synthesis gas.

The synthesis gas generated by the waste gas reformer 100 includes carbon monoxide, carbon dioxide, and hydrogen. The synthesis gas generated in the waste gas reformer 100 includes approximately 0% to 70% hydrogen, 0% to 50% carbon monoxide, 0% to 20% carbon dioxide, and 5% or less methane. Specifically, a concentration of carbon dioxide in the synthesis gas may be at least 5%, and a molar ratio of carbon monoxide to carbon dioxide may be approximately 1:1. For example, the synthesis gas composition may include 65% to 67% hydrogen (H₂), 15% carbon monoxide (CO), 15% to 17% carbon dioxide (CO₂), and 0.3% to 0.4% methane (CH₄).

The synthesis gas is supplied to the methanol production reactor 200, which is configured to produce methanol utilizing synthesis gas containing carbon monoxide, hydrogen, and a high-concentration carbon dioxide. Accordingly, the system enables methanol production under high carbon dioxide concentration conditions without requiring an upstream separation or removal process to eliminate carbon dioxide from the synthesis gas prior to the reaction.

In the methanol production reactor 200, carbon monoxide and carbon dioxide are each reacted with hydrogen to produce methanol. Specifically, the methanol production reactor 200 includes a first reaction zone 220 and a second reaction zone 240 disposed therein. The synthesis gas is supplied to the first reaction zone 220 and reacted in the presence of a first catalyst to generate methanol. Subsequently, unreacted gas from the first reaction zone 220 is supplied to the second reaction zone 240 and reacted in the presence of a second catalyst to generate methanol. As such, the methanol production reactor 200 may be configured as a single reactor containing both the first reaction zone 220 and the second reaction zone 240.

In the first reaction zone 220, carbon monoxide and hydrogen react in the presence of the first catalyst to produce methanol primarily according to Chemical Formula (1), and in the second reaction zone 240, carbon dioxide and hydrogen react in the presence of the second catalyst to produce methanol primarily according to Chemical Formula (2).

Chemical Formula (1) - CO + 2H₂ → CH₃OH

Chemical Formula (2) - CO₂ + 3H₂ → CH₃OH + H₂O

As a result of the primary reaction between carbon monoxide and hydrogen in the first reaction zone 220, the unreacted gas supplied to the second reaction zone 240 is characterized by a higher concentration of carbon dioxide and hydrogen relative to carbon monoxide.

To adjust the synthesis gas discharged from the waste gas reformer 100 to a pressure and temperature suitable for methanol production, a first compressor 120 and a heat exchanger 140 are disposed between the waste gas reformer 100 and the methanol production reactor 200.

The first compressor 120 is configured to compress the synthesis gas and supply the compressed synthesis gas to the first reaction zone 220. In one or more exemplary embodiments, the synthesis gas is pressurized by the first compressor 120 to a pressure ranging from approximately 20 bar to 100 bar.

The heat exchanger 140 is configured to heat the synthesis gas and supply the heated synthesis gas to the first reaction zone 220. In one or more exemplary embodiments, the synthesis gas is heated by the heat exchanger 140 to a temperature ranging from approximately 150°C to 300°C.

In the present embodiment, the first compressor 120 and the heat exchanger 140 are configured to provide the synthesis gas to the first reaction zone 220 at a pressure of approximately 70 bar and a temperature of approximately 250°C.

Further, the unreacted gas discharged from the first reaction zone 220 is supplied to the second reaction zone 240. In a preferred configuration, methanol and water mixed in the unreacted gas discharged from the first reaction zone 220 are removed via a separation process, and a purified unreacted gas is provided to the second reaction zone 240.

The system further includes a second condenser 300 configured to condense methanol and water from the gas discharged from the first reaction zone 220. The second condenser 300 is in fluid communication with the second reaction zone 240, such that only the unreacted gas remaining after the condensation and separation of methanol and water is supplied to the second reaction zone 240. The methanol and water condensed in the second condenser 300 are subsequently delivered to the distillation column 500 for recovery of high-concentration methanol.

As described above, by condensing and separating methanol and water as intermediate products from the gas discharged from the first reaction zone 220 and supplying only the unreacted gas to the second reaction zone 240, the reaction efficiency in the second reaction zone 240 can be improved. Consequently, the required volume of the second reaction zone 240 can be reduced relative to the first reaction zone 220.

In one or more exemplary embodiments, the heat exchanger 140 may use the gas discharged from the first reaction zone 220 as a heat source. Accordingly, the gas discharged from the first reaction zone 220 may be supplied to the second condenser 300 after its temperature is lowered while heating the synthesis gas, thereby improving the efficiency of the second condenser 300.

Downstream of the methanol production reactor 200, a first condenser 400 is provided to condense methanol and water from the gas discharged from the methanol production reactor 200, e.g., from the second reaction zone 240. The methanol and water condensed in the first condenser 400 may be delivered to the distillation column 500, where they are processed to recover high-purity methanol.

In one or more exemplary embodiments, the methanol and water condensed in the second condenser 300 and the methanol and water condensed in the first condenser 400 are merged and collected in a merge drum 700 before being delivered to the distillation column 500. However, the present disclosure is not limited thereto, in alternative embodiments, methanol and water condensed from the second condenser 300 and the first condenser 400 may be delivered to the distillation column 500 as independent feed streams.

In one or more exemplary embodiments, the heat exchanger 140 may use the gas discharged from the second reaction zone 240 as a heat source. Accordingly, the gas discharged from the second reaction zone 240 may be supplied to the first condenser 400 after its temperature is lowered while heating the synthesis gas, thereby improving the efficiency of the first condenser 400.

Referring to FIG. 1, the heat exchanger 140 is illustrated at three distinct positions for clarity of the process flow, but these illustrations may represent a single, integrated heat exchanger 140. Alternatively, the system may comprise a plurality of discrete heat exchangers. For example, a first heat exchanger may be configured to heat the synthesis gas supplied to the first reaction zone 220 using the gas discharged from the first reaction zone 220 as a heat source, and a second heat exchanger may be separately provided to heat the synthesis gas supplied to the first reaction zone 220 using the gas discharged from the second reaction zone 240 as a heat source.

The gas discharged from the first condenser 400, following the condensation and separation of methanol and water, includes hydrogen, carbon monoxide, and carbon dioxide unreacted in the methanol production reactor 200. The gas discharged from the first condenser 400 may be directed to a stack.

The gas separator 600 is configured to recover hydrogen, or a mixture of hydrogen and carbon monoxide, as a recycle gas from the gas discharged from the first condenser 400. While the entire volume of the gas discharged from the first condenser 400 may be supplied to the gas separator 600, in one or more exemplary embodiments, a portion of the gas discharged from the first condenser 400 is branched and supplied to the gas separator 600.

In one or more exemplary embodiments, a flow control valve 420 may be disposed within the branched line to control a flow rate of the gas supplied to the gas separator 600. The flow control valve 420 is configured to adjust the volume of the branched gas stream to provide an optimal gas flow rate to the gas separator 600.

For example, the gas separator 600 comprises a pressure swing adsorption (PSA) apparatus configured to isolate hydrogen. However, the present disclosure is not limited thereto, the gas separator 600 may alternatively comprise a hydrogen separation membrane or other suitable gas-selective separation structures. Accordingly, hydrogen separated in the gas separator 600 may be returned to the system as a recycle gas for methanol synthesis. Conversely, the remaining components, such as carbon monoxide and carbon dioxide, that are rejected by the gas separator 600 are discharged to a stack.

The recycle gas separated in the gas separator 600 is merged with the synthesis gas supplied to the first reaction zone 220. Specifically, the recycle gas is merged with the synthesis gas at a junction located upstream of the heat exchanger 140, such that the synthesis gas merged with the recycle gas is supplied to the heat exchanger 140.

A second compressor 620 may be further provided to compress the recycle gas separated in the gas separator 600 and merge the compressed recycle gas with the synthesis gas supplied to the first reaction zone 220. Because the pressure of the recycle gas is reduced during the separation process, the second compressor 620 is configured to pressurize the recycle gas to a pressure substantially equal to the pressure of the synthesis gas with at the point of merging.

In one or more exemplary embodiments, it is preferable that a methanol synthesis ratio R = (H₂-CO₂)/(CO+CO₂) of the synthesis gas supplied to the first reaction zone 220 after merging with the recycle gas is within a range of approximately 1.5 to 2.5. Accordingly, after the methanol production reaction, hydrogen is used as a recycle gas to adjust the methanol synthesis ratio R of the synthesis gas supplied to the first reaction zone 220 to a range of 1.5 to 2.5, specifically to 2.0, thereby maximizing methanol production efficiency.

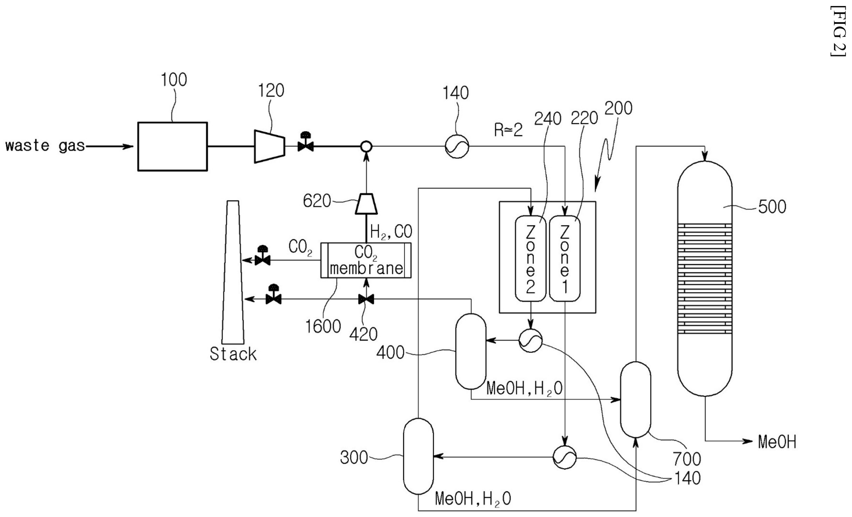

A methanol production system according to a second exemplary embodiment will be described with reference to FIG. 2.

Referring to FIG. 2, the methanol production system includes a waste gas reformer 100, a first compressor 120, a heat exchanger 140, a methanol production reactor 200, a second condenser 300, a first condenser 400, a distillation column 500, a gas separator 1600, and a second compressor 620. The methanol production system of FIG. 2 is substantially similar to the methanol production system of FIG. 1, with the exception of the configuration of the gas separator 1600. Accordingly, for the sake of brevity, only the features of the gas separator 1600 that differ from the first embodiment will be described.

In the present embodiment, the gas separator 1600 comprises a carbon dioxide separation membrane configured to selectively separate carbon dioxide. However, the present disclosure is not limited thereto, and the gas separator 1600 may comprise various other structural devices suitable for carbon dioxide isolation. In this configuration, carbon dioxide is separated and removed by the gas separator 1600, while the remaining hydrogen and carbon monoxide may be recycled for methanol production as a recycle gas. The carbon dioxide separated by the gas separator 1600 is subsequently discharged to a stack.

Similarly, the recycle gas separated by the gas separator 1600 is merged with the synthesis gas supplied to the first reaction zone 220.

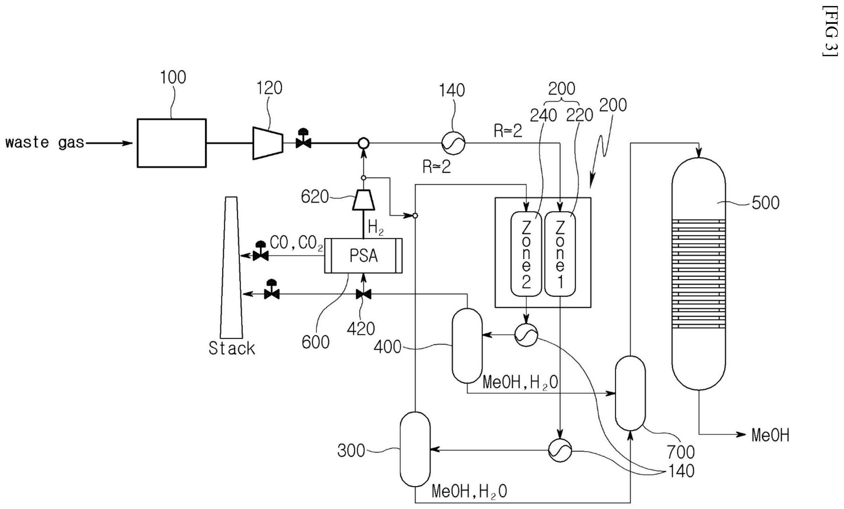

A methanol production system according to a third exemplary embodiment will be described with reference to FIG. 3.

Referring to FIG. 3, the methanol production system includes a waste gas reformer 100, a first compressor 120, a heat exchanger 140, a methanol production reactor 200, a second condenser 300, a first condenser 400, a distillation column 500, a gas separator 600, and a second compressor 620. The methanol production system of FIG. 3 differs from the methanol production system of FIG. 1 in that the recycle gas is merged at multiple injection points. For example, the recycle gas is merged with the synthesis gas supplied to the first reaction zone 220 and is further merged with the unreacted gas supplied to the second reaction zone 240.

More specifically, a portion of the recycle gas separated by the gas separator 600 is diverted and merged with the unreacted gas supplied to the second reaction zone 240. In this configuration, the recycle gas is merged with the synthesis gas supplied to the first reaction zone 220, and a portion of the recycle gas is branched and merged with the unreacted gas supplied to the second reaction zone 240.

In one or more exemplary embodiments, it is preferable that a methanol synthesis ratio R = (H₂-CO₂)/(CO+CO₂) of the unreacted gas supplied to the second reaction zone 240 after merging with the recycle gas is maintained within a range of approximately 1.5 to 2.5. Accordingly, after the methanol production reaction, hydrogen is used as a recycle gas to adjust the methanol synthesis ratio R of the synthesis gas supplied to the first reaction zone 220, as well as the methanol synthesis ratio R of the unreacted gas supplied to the second reaction zone 240, to a range of 1.5 to 2.5, specifically to 2.0, thereby maximizing methanol production efficiency.

While one or more exemplary embodiments have been described with reference to the accompanying drawings, it will be apparent to those skilled in the art that various variations and modifications may be made by adding, changing, or removing components without departing from the spirit and scope of the disclosure as defined in the claims, and these variations and modifications fall within the spirit and scope of the disclosure as defined in the appended claims. Therefore, the description of the exemplary embodiments should be construed in a descriptive sense and not to limit the scope of the claims, and many alternatives, modifications, and variations will be apparent to those skilled in the art.

## Claims

1. A methanol production system comprising:
a waste gas reformer configured to reform a waste gas comprising a hydrocarbon (CxHy) gas and carbon dioxide to generate a synthesis gas containing hydrogen, carbon monoxide, and carbon dioxide;
a methanol production reactor including a first reaction zone containing a first catalyst, the first reaction zone configured to receive the synthesis gas and facilitate a reaction of the synthesis gas to generate methanol, and a second reaction zone containing a second catalyst, the second reaction zone configured to receive unreacted gas from the first reaction zone and facilitate a reaction of the unreacted gas to generate methanol;
a first condenser configured to condense and separate methanol and water from gas discharged from the methanol production reactor; and
a gas separator configured to separate a recycle gas comprising hydrogen, or a mixture of hydrogen and carbon monoxide, from gas discharged from the first condenser.

2. The methanol production system according to claim 1,
wherein the gas separator comprises a pressure swing adsorption (PSA) apparatus or a hydrogen-selective membrane configured to separate hydrogen, and the hydrogen separated in the gas separator is recycled for methanol production as the recycle gas.

3. The methanol production system according to claim 1,
wherein the gas separator comprises a carbon dioxide-selective membrane configured to separate carbon dioxide, and hydrogen and carbon monoxide remaining after the separation of carbon dioxide by the gas separator are recycled for methanol production as the recycle gas.

4. The methanol production system according to claim 1,
wherein the recycle gas separated in the gas separator is merged with the synthesis gas supplied to the first reaction zone.

5. The methanol production system according to claim 4,
wherein a portion of the recycle gas separated in the gas separator is merged with the unreacted gas supplied to the second reaction zone.

6. The methanol production system according to claim 1, further comprising:
a second condenser configured to condense methanol and water from gas discharged from the first reaction zone, and
wherein only the unreacted gas discharged from the second condenser after methanol and water are condensed and separated is supplied to the second reaction zone.

7. The methanol production system according to claim 4, further comprising:
a first compressor configured to compress the synthesis gas and deliver the compressed synthesis gas to the first reaction zone, and
wherein the first compressor is configured to pressurize the synthesis gas to a pressure within a range of approximately 20 bar to 100 bar.

8. The methanol production system according to claim 7, further comprising:
a second compressor configured to compress the recycle gas separated by the gas separator and merge the compressed recycle gas with the synthesis gas supplied to the first reaction zone.

9. The methanol production system according to claim 4, further comprising:
a heat exchanger configured to heat the synthesis gas and supply the heated synthesis gas to the first reaction zone, and
wherein the synthesis gas is heated to a temperature within a range of approximately 150°C to 300°C by the heat exchanger.

10. The methanol production system according to claim 9,
wherein the heat exchanger uses the gas discharged from the first reaction zone or the second reaction zone as a heat source.

11. The methanol production system according to claim 9,
wherein the recycle gas separated by the gas separator is merged with the synthesis gas at a position upstream of the heat exchanger.

12. The methanol production system according to claim 1,
wherein a portion of the gas discharged from the first condenser after methanol and water are condensed and separated is branched and supplied to the gas separator, and
the methanol production system further comprising:
a flow control valve configured to control a flow rate of the gas branched and supplied to the gas separator.

13. The methanol production system according to claim 1,
wherein the methanol production reactor is configured as a single reactor in which the first reaction zone and the second reaction zone are disposed therein.

14. The methanol production system according to claim 1,
wherein the waste gas supplied to the waste gas reformer has a molar ratio of methane to carbon dioxide of 7:3, or a methane molar ratio lower than 7:3.

15. The methanol production system according to claim 1,
wherein the synthesis gas generated by the waste gas reformer includes 0% to 70% hydrogen, 0% to 50% carbon monoxide, 0% to 20% carbon dioxide, and 5% or less methane.
